# EUROPÄISCHE PATENTSCHRIFT

(11) **EP 2 911 595 B1**
(45) Veröffentlichungstag und Bekanntmachung des Hinweises auf die Patenterteilung: **27.12.2017**
(21) Anmeldenummer: 13783307.5
(22) Anmeldetag: 23.10.2013
(51) Int. Cl.: A61B 17/11, A61B 18/14, A61B 17/115, A61B 17/00, A61B 17/072, A61B 18/00

(54) **ANASTOMOSEINSTRUMENT MIT SCHWENKBAREM AMBOSS**
ANASTOMOSIS INSTRUMENT WITH PIVOTABLE ANVIL
INSTRUMENT D'ANASTOMOSE À ENCLUME PIVOTANTE

(30) Priorität: 29.10.2012 DE 102012110312
(43) Veröffentlichungstag der Anmeldung: 02.09.2015
(73) Patentinhaber: Aesculap AG, 78532 Tuttlingen (DE)
(72) Erfinder: HAFNER, Nikolaus, 78532 Tuttlingen (DE); ROTHWEILER, Christoph, 78166 Donaueschingen (DE)
(74) Vertreter: Winter, Brandl, Fürniss, Hübner, Röss, Kaiser, Polte - Partnerschaft mbB
(86) Internationale Anmeldenummer: PCT/EP2013/072157
(87) Internationale Veröffentlichungsnummer: WO 2014/067829

(56) Entgegenhaltungen:
- EP-A1- 1 857 058
- EP-A2- 0 698 376
- DE-U1-202010 013 151
- DE-U1-202012 100 197
- US-A- 4 566 620

## Beschreibung

Die Erfindung betrifft ein chirurgisches Instrument, das zur Herstellung einer Anastomose geeignet ist mit einem in einem Instrumentenschaft axial verschiebbar einführbaren Kopfstückschaft, an dessen distalem Endabschnitt ein platten- oder ambossartiges Kopfstück axial verschiebbar und über einen Querstift zwischen einer Bearbeitungsposition und einer Entnahmeposition schwenkbar gelagert ist.

Das Kopfstück kann auch als Amboss bezeichnet werden, genauso wie der Kopfstückschaft als Kopfschaft, Mittelstange oder Mittelstab bezeichnet werden kann.

Solche Anastomoseinstrumente sind bspw. aus der EP 1 857 058 B1 bekannt, die die Merkmale des Oberbegriffs von Anspruch 1 offenbart. Dort ist eine Neigungsambossanordnung offenbart, die zur Verwendung mit einem kreisförmigen Anastomose-Klammergerät geeignet ist. Diese Vorrichtung umfasst einen Mittelstab, eine Kopfanordnung (Amboss) mit einem Gehäuse, einen Absatz, eine Ambossplatte mit Klammerdeformationsbeulen und ein Schneidringstützelement. Dabei ist die Kopfanordnung schwenkbar an dem Mittelstab befestigt und schwenkbar in Bezug auf den Mittelstab zwischen einer nicht geneigten Position senkrecht zu der Achse des Mittelstabes und einer geneigten Position schwenkbar. Das Schneidringstützelement ist um den Absatz positioniert und wird entlang des Absatzes aus einer ersten Position, in der ein Abschnitt des Stützelementes positioniert ist, um eine Schwenkbewegung der Kopfanordnung von der nicht geneigten Position zu der geneigten Position zu verhindern, in eine zweite Position bewegt, in der das Stützelement positioniert ist, um eine Schwenkbewegung der Kopfanordnung in Bezug auf den Mittelstab aus der nicht geschwenkten Position in die geschwenkte Position zu erlauben, wenn das Klammergerät ausgelöst wird. Die Kopfanordnung enthält ferner ein Riegelelement, das positioniert ist, um eine Zurückbewegung des Stützelementes aus der zweiten Position in die erste Position zu verhindern, wobei insbesondere das Riegelelement ein Schwenknockenriegelelement ist. Eine solche Vorrichtung wird bspw. bei der Herstellung einer chirurgischen End-zu-End Anastomose verwendet. Natürlich kann ein solches Instrument auch bei Seit-zu-Seit-Anastomosen, Seit-zu-End-Anastomosen und End-zu-Seit-Anastomosen verwendet werden, insbesondere im Magen, Speiseröhren- und Darmbereich.

Zu aufwändigen Handnähten, die eingesetzt werden, um zwei Teile bspw. des Darms miteinander zu verbinden, kann das Instrument der EP 1 857 058 B1 als dazu alternatives zirkuläres Klammernahtinstrument eingesetzt werden.

Bei solchen Klammernahtinstrumenten wird der Amboss axial gegen ein Klammermagazin stirnseitig gedrückt, wobei Körpergewebe dazwischen eingeklemmt wird. Klammern werden anschließend durch das eingeklemmte Gewebe, also bspw. durch den Darm gestochen und am Amboss (bzw. am Schneidringstützelement) umgeformt, so dass eine axiale Verbindung zwischen zwei schlauchartigen Enden des Darms zustande kommt. Um die Kontinuität des Hohlorgans wieder herzustellen, wird bspw. mit einer runden Klinge (Kreisringklinge) der axiale Durchgang im Klammerbereich ausgestanzt, wofür der Amboss als axiales Widerlager für die im Klammermagazin gelagerte Kreisklinge dient.

Bei der Entfernung des Instruments, d.h. beim Hindurchziehen (Zurückziehen / Entfernen) des Ambosses durch die Klammernaht sollen die verbundenen Darmbereiche nicht zu stark gedehnt werden, da dies zu einer Schädigung der gerade geschaffenen Klammernaht führen kann. Aus diesem Grund wird häufig der Amboss nach dem Klammerprozess durch einen Federmechanismus abgeklappt/verschwenkt. Der Amboss bzw. das Kopfstück ist über einen Kopfstückschaft, eine Mittelstange oder einen Mittelstab gelagert und wird im besagten Betätigungsfall in eine Entnahmestellung gebracht. Diese Entnahmestellung kann auch als Entnahmeposition bezeichnet werden. In dieser Entnahmestellung / -position weist das Anastomoseinstrument bzw. der Amboss (das Kopfstück) eine kleinere Projektionsfläche auf, als in der Bearbeitungsstellung (bei Betrachtung in Längsrichtung des Instruments). Der Abklappvorgang wird in der Regel durch den Vorschub der Klinge aktiviert, meistens dadurch, dass ein Rückhalteelement, wie eine verformbare Scheibe dauerhaft deformiert, d.h. partiell zerstört wird.

Durch das Abklappen des Kopfstücks (Amboss / Ambosskopf), wird der bei Betrachtung in Richtung der Längsachse des Instruments wirkende Umfang so reduziert, dass die gesetzten Klammerreihen bei der Entnahme des Instruments nicht überdehnt werden. Die Gefahr einer Leckage der Anastomose wird durch den in die Entnahmeposition verbrachten, abwinkelbaren Amboss reduziert, da die mechanische Belastung der Naht beim Entfernen des Instruments deutlich geringer ausfällt.

Bei den bekannten, mechanische Klammern verwendenden Instrumenten sowie bei einer manuellen Handnaht, wird das Gewebe stellenweise perforiert. Diese Perforationen sind potenzielle Leckage-Stellen und auch Stellen, die sich entzünden können. Dies gilt es, bei einem Anastomoseinstrument künftig zu vermeiden.

Aus dem Stand der Technik, etwa der DE 20 2010 013 151 U1 ist auch ein diesbezüglich verbessertes, chirurgisches System zum Verbinden von Körpergewebe, umfassend ein chirurgisches Instrument mit einer Verbindungseinrichtung zum Verbinden von Körpergewebe, bekannt. Dabei umfasst die Verbindungseinrichtung zwei relativ zueinander bewegbare Werkzeugelemente. Das Instrument hat ferner eine Schneideinrichtung mit einem Schneidelement zum Durchtrennen von Gewebe, wobei das Schneidelement relativ zu zumindest einem der Werkzeugelemente bewegbar angeordnet ist. Das Schneidelement hat eine Schneidkante, welche eine relativ zu einer vom Instrument im Bereich der Verbindungseinrichtung definierten Längsachse geneigte Schneidebene definiert. Dieses chirurgische System verwendet eine mit elektrischem Strom agierende Hochfrequenz-Schneideinrichtung (HF-Schneideinrichtung). Monopolare und bi-polare Schneideinrichtungen werden dort ebenso offenbart.

Mit diesem System kann auf Klammern verzichtet werden, so dass die sog. "Tissue Fusion Technology" (TFT) anwendbar ist. Hierbei werden unterschiedliche oder auch gleiche Gewebearten durch den Einsatz von HF-Energie, vorzugsweise bi-polar, miteinander verbunden. Da bei dieser Technologie die Gewebeschichten nicht durch Klammern miteinander verbunden werden, ist es von Vorteil, wenn auch hier keine großen Kräfte auf die Anastomose / Anastomosennaht wirken, wenn das Anastomoseinstrument entnommen wird. Es sind daher dort ebenfalls abwinkelbare Elektrodenplatten vorgesehen, um eine Schädigung der Anastomose beim Herausziehen des Instruments zu vermeiden.

Leider sind die aus dem Stand der Technik bekannten Instrumente zum Abklappen des Kopfstücks (Amboss) meist nicht reversibel ausgestaltet, so dass ein schnelles und problemloses Wiedereinsetzen ein und desselben Instrumentes im Rahmen einer medizinischen Behandlung bspw. am menschlichen Körper nicht möglich ist. Hier gilt es, eine Verbesserung zur Verfügung zu stellen. Auch gibt es bisher keine Möglichkeit zu erkennen, ob der Abklappvorgang korrekt durchgeführt wurde, also ob das Kopfstück korrekt in die Entnahmestellung verbracht wurde.

Es ist daher eine der Aufgaben der Erfindung, diesen Zustand zu ändern und vorzugsweise dem Operateur eine Rückmeldung zur Verfügung zu stellen.

Da die bisher bekannten Instrumente meist eine aufwändige Mechanik beinhalten und sehr viele Bauteile einsetzen, sind die Herstellung, ggf. die Reinigung, die Wartung und der Betrieb sehr aufwändig und ziehen erhebliche Kosten nach sich. Auch hier soll eine Verbesserung erreicht werden.

Letztlich soll auch das Verbringen des Kopfstücks in eine Entnahmestellung derart kontrolliert stattfinden, dass die auf die am Hohlorgan vorhandene versiegelte Fläche wirkenden Kräfte, die beim Entfernen des Kopfstücks entstehen, reduziert werden.

Diese Aufgabe wird durch ein Anastomoseinstrument mit den Merkmalen des Anspruchs 1 gelöst. Diese Aufgabe wird erfindungsgemäß insbesondere dadurch gelöst, dass das Kopfstück (der Amboss) an seinem Kopfstückschaft aus einer ersten, proximalen zurückgeschobenen Raststellung, in welcher der Kopfstückschaft formschlüssig mit dem Kopfstück gekoppelt ist, um das Kopfstück in der Bearbeitungsposition (Bearbeitungsstellung) unverschwenkbar zu fixieren, in eine zweite, distal vorgeschobene Raststellung verlagert oder verschoben ist (verlagerbar oder verschiebbar ist), in welcher der Formschluss freigegeben (entriegelt / aufgelöst / beseitigt) ist, um das Verschwenken (Verdrehen / Rotieren / Abklappen) des Kopfstücks um ein Scharnier oder Gelenk in die Entnahmeposition (Entnahmestellung) zu ermöglichen. In der Bearbeitungsposition (Bearbeitungsstellung) findet ein verbindendes Bearbeiten zweier Teile des Hohlorgans (Darm), etwa über das Nutzen von Klammern und/oder von Elektrizität (HF) statt. In der Entnahmeposition ist das Anastomoseinstrument so verschwenkt, dass es nahezu widerstandsfrei aus dem Hohlorgan entfernbar ist.

Das Kopfstück ist somit relativ zum Kopfstückschaft bei Lösen einer Verrastung oder Verriegelung in einen Zustand bringbar, in dem ein Verschwenken bspw. automatisch über eine vorgespannte Feder erreicht werden kann. Ist das Kopfstück in die zweite, distal vorgeschobene Raststellung verlagert, oder verschoben, bspw. durch Einwirkung aufgrund eines Messers (wie einer Kreisklinge) oder gewolltes Betätigen des Schaftes über einen vordefinierten Widerstand hinaus, so schwenkt das Kopfstück selbsttätig, nahezu automatisch von der Bearbeitungsposition in die Entnahmeposition sobald das Instrument wieder weit genug geöffnet wurde. Befindet sich das Kopfstück in einer Entnahmeposition, so liegt sein proximales Ende, an dem etwaige Taschen zur Aufnahme von Klammern oder (alternativ oder kumulativ) eine oder mehrere Elektroden vorhanden sind, in einer Ebene, durch die die Längsachse des Kopfstückschaftes quer verläuft, nämlich in einem (vor-)bestimmten Winkel a.

Beim Verbringen des Kopfstücks von der ersten Raststellung, in der der Formschluss zwischen dem Kopfstück und dem Kopfstückschaft derart vorhanden ist, so dass ein Verschwenken des Kopfstücks (um eine zur Längsachse quer / orthogonal abstehende Schwenkachse) relativ zum Kopfstückschaft ausgeschlossen ist, in eine distal weiter beabstandete zweite Raststellung, wird ein Verschwenken erst ermöglicht. Der bis dahin zwischen dem Kopfstück und dem Kopfstückschaft vorliegende Formschluss ist dann aufgelöst. Ein Herausziehen oder Herausschieben des Kopfstückes aus dem Kopfstückschaft stellt das Verbringen des Kopfstückes in diese zum Verschwenken notwendige Ausgangslage sicher.

Die auf die versiegelte Fläche im Darmbereich wirkenden Kräfte, welche beim Herausziehen des Kopfstückes, bspw. mit einer Elektrodenplatte aus dem Hohlorgan entstehen, werden somit reduziert. Das Verschwenken des Kopfstückes kann kontrolliert stattfinden, genauso wie das Entnehmen des Anastomoseinstruments aus dem Hohlorgan (bspw. dem Darm) kontrolliert erfolgen kann.

Der Kern der vorliegenden Erfindung betrifft zusammengefasst einen Schwenkmechanismus für den Amboss eines bipolaren Anastomoseinstruments. Der Schwenkmechanismus sieht erfindungsgemäß zwei voneinander entkoppelte Bewegungsmuster vor: Zunächst ist der Amboss bezüglich eines Kopfstückschafts axial verschiebbar, um aus einer proximal zurückgezogenen Position, in welcher eine Formschlussverbindung den Amboss starr am Kopfstückschaft hält in eine distal vorgeschobene Position zu bringen, in welcher die Formschlussverbindung gelöst wird. Des Weiteren ist der Amboss ausschließlich in der distal vorgeschobenen Position bezüglich des Kopfstückschafts verschwenkbar an dem Kopfstückschaft gelagert.

Vorteilhafte Ausführungsbeispiele werden in den Unteransprüchen beansprucht und sind nachfolgend näher erläutert.

So ist es von Vorteil, wenn das Kopfstück auf seiner proximalen, dem Kopfstückschaft zugewandten Seite eine Elektrode aufweist, die vorzugsweise in einer Elektrodenplatte gehalten ist und ringförmig ausgebildet ist und/oder eine Vielzahl von Taschen zum Bearbeiten/Umbiegen von Klammern vorhanden ist. Die Elektrode bzw. die Elektroden können dabei auch die Taschen bilden. Die Taschen sind dann nach Art von Kammern ausgebildet, in die die Klammern eindringen und dort umgebogen werden. Es kann auf diese Weise unter Zuhilfenahme von Klammern die Verbindung der Gewebeteile des Darms erreicht werden oder aber auf die Klammern verzichtet werden und nur über das verschweißende Einwirken der Elektrizität, die über die Elektrode aufgebracht wird, das Verbinden erzielt werden. Auch eine Kombination dieser zwei Möglichkeiten ist denkbar.

Es ist zweckmäßig, wenn ein Querstift welcher das Kopfstückscharnier bildet, in einem Längsschlitz im Kopfstückschaft zwischen den beiden Raststellungen verschieblich, hin und her bewegbar geführt ist. Der Längsschlitz agiert dann als Führung/Kulisse, wobei sich der Längsschlitz bis zum distalen Ende des Kopfstückschaftes erstrecken und sich dort stirnseitig öffnen kann und auf diese Weise eine federnde Federgabel-förmige Ausgestaltung des Kopfstückschaftes im Bereich der Führung (in Querrichtung / in Radialrichtung) sicherstellt.

Dabei ist es von Vorteil, wenn der Kopfstückschaft im Bereich des Längsschlitzes in Querrichtung federelastisch ausgebildet ist, da dadurch der Querstift in bspw. einer ersten Querbohrung (1. Raststellung im Längsschlitz) gehalten ist und erst nach Aufspreizung des Kopfstückschaftes, in eine zweite dem distalen Ende, von der ersten Querbohrung aus gesehen, nähere zweite Querbohrung (2. Raststellung im Längsschlitz) verbracht werden kann. Es muss also dezidiert ein Widerstand überwunden werden, damit der Querstift aus seiner ersten Raststellung in seine zweite Raststellung gelangen kann. Dazu sollte mehr Kraft als beim normalen Benutzen des Instruments in seiner Bearbeitungsposition aufgebracht werden. In anderen Worten ausgedrückt, sollte die Kraft, die erforderlich ist, um den Querstift aus seiner ersten Rastposition entgegen der Federkraft des geschlitzten Kopfstückschafts zu verschieben, größer sein als alle jene Kräfte, die beim Standardgebrauch des Instruments auf den Querstift einwirken. Ein unbeabsichtigtes Überspringen des Querstiftes von der ersten Raststellung in die zweite Raststellung ist dadurch vermeidbar. Dadurch kann ein unbeabsichtigtes Abklappen / Verschwenken des Kopfstücks /Amboss verhindert werden. Natürlich kann die Auslösekraft auch schwächer eingestellt sein als die maximal zu erwartenden Bearbeitungskräfte. In diesem Fall wird ein Auslösen der ersten Raststellung bereits im Standardbetrieb (jene Kraft, die für eine ausreichende Gewebeverschweißung durch Koagulation bekanntermaßen erforderlich ist) bezweckt, um die Entnahme des Instruments unmittelbar mit Beendigung der Gewebeverschweißung zu ermöglichen.

Es ist dann von Vorteil, wenn das Kopfstück in der zweiten, distal vorgeschobenen Raststellung um den Querstift, der vorzugsweise orthogonal zur Längsachse des Kopfstücks ausgerichtet ist, in die Entnahmeposition verschwenkbar ist. Der Querstift kann dann als Schwenklager dienen, um das das Kopfstück nach Auflösung des Formschlusses in der ersten Rastposition zwischen der Außenseite des Kopfstückschaftes und einer Innenseite des Kopfstücks rotiert.

Es ist auch zweckmäßig, wenn ein Federelement, wie eine mechanische Feder, so am Kopfstück und dem Kopfstückschaft ggf. angebracht ist, dass ein Verschwenken des Kopfstücks bei Verlagerung des Kopfstücks von der ersten Raststellung in die zweite Raststellung erzwungen wird. Eine Art Automatik ist dann realisierbar, so dass nach Verbringen des Kopfstücks in die distal fernere Position (Querstift dann in der zweiten Raststellung), selbsttätig ein Abklappen oder Verschwenken des Kopfstücks in die Entnahmeposition stattfinden kann.

Als mechanisch besonders sinnvoll und einfach zu montieren hat es sich herausgestellt, die Feder dezentral, von der Längsachse des Kopfstückschafts quer beabstandet (vorzugsweise orthogonal von der Längsachse des Kopfstückschafts beabstandet) im Kopfstückschaft zu lagern, so dass der Längsschlitz zentral angeordnet sein kann. Dadurch kann das Kopfstück auch symmetrisch ausgeformt sein.

Wenn die Feder im Kopfstück oder im Kopfstückschaft ferner verschieblich gelagert ist, vorzugsweise in einer schaftartigen Ausnehmung oder einem ähnlichen Hohlraum, so hindert die Feder nicht das axiale Verschieben des Kopfstücks aus der ersten Raststellung in die zweite Raststellung bei gleichzeitigem Verschwenken des Kopfstücks um den Stift, ausgelöst durch die (Biege-)Feder.

Ein vorteilhaftes Ausführungsbeispiel ist auch dadurch gekennzeichnet, dass die Feder als Blattfeder, Schenkelfeder oder Druckfeder (etwa als Schraubenfeder) ausgebildet ist, die vorzugsweise in der Entnahmeposition in einem Zustand geringerer Spannung als in der Bearbeitungsposition befindlich ist, weiter vorzugsweise in der Entnahmeposition im Wesentlichen entspannt ist.

Damit ein unbeabsichtigtes Auslösen, also Verschwenken des Kopfstückschaftes, vermieden wird, ist es von Vorteil, wenn der Querstift und der Längsschlitz so aufeinander abgestimmt sind, dass beim Verbringen des Kopfstücks von der ersten Raststellung in die zweite Raststellung ein vorbestimmter Widerstand zu überwinden ist, wobei zum Überwinden des Widerstands weiter vorzugsweise eine größere Kraft benötigt ist, als die Kraft, die bei einem normalen, ein Abschwenken des Kopfstücks nicht hervorrufendes Betätigen des Anastomoseinstruments vorliegt, etwa beim Durchtrennen von Gewebe durch ein im Instrument integriertes Messer oder bei Verschiebung des Kopfstückschafts zur Anpassung des Instruments an die Dicke des zu bearbeitenden Gewebes sowie zur Druckbeaufschlagung des Patientengewebes während der Bestromung und/oder Klammerung.

Ferner ist es von Vorteil, wenn die Elektrode mittels der Feder (z.B. mittels der Blattfeder) elektrisch kontaktiert ist. Zusätzlich oder alternativ kann auch eine Litze zur elektrischen Kontaktierung genutzt werden.

Mit anderen Worten, wird grundsätzlich eine abwinkelbare Elektrodenplatte (Kopfstück/Amboss) mit einem Schaft zur Aufnahme im Schaft eines Anastomoseinstruments vorgestellt. Der Kopfstückschaft, der im Instrumentenschaft längsverschiebbar gelagert ist enthält an seinem distalen Endabschnitt zwei übereinander angeordnete Quer-Durchgangs-Bohrungen, die durch einen verjüngten Zwischenbereich in Verbindung stehen. Oberhalb der oberen Bohrung ist der Kopfstückschaft ebenfalls geschlitzt/gegabelt ausgebildet. Dabei sei an dieser Stelle darauf hingewiesen, dass der Kopfstückschaft endseitig nicht notwendiger Weise gegabelt sein muss, sondern auch endseitig geschlossen sein kann, d. h. der Längsschlitz ist stirnseitig nicht offen sondern geschlossen. In diesem Fall muss das Schaftmaterial (z.B. PEEK) ausreichend elastisch sein, um ein radiales Aufweiten des Schlitzes durch den Querstift bei dessen Längsverschiebung zu ermöglichen.

In der Grundstellung, also der Bearbeitungsposition, ist der Schaft rechtwinklig zur Elektrodenplatte angeordnet. In dieser Position ist ein Abwinkeln der Elektrodenplatte aufgrund des Formschlusses zwischen der Elektrodenplatte und dem distalen Ende des Kopfstückschaftes nicht möglich, da der Kopfstückschaft so an der Elektrodenplatte anliegt, dass er sich nicht drehen/relativ verschwenken kann.

Wird nun eine vorbestimmte Kraft in axialer Richtung des Kopfstückschafts aufgebracht, so wird dieser sich in derselben Richtung mit bewegen. Dabei wird der Querstift in die zweite Bohrung, die oberhalb der ersten liegt, einschnappen. In dieser zweiten Position ist die Elektrodenplatte drehbar. Durch das Einwirken bspw. einer Federkraft, wird die Elektrodenplatte abgewinkelt und in die Entnahmeposition verbracht, sobald das Instrument wieder weit genug geöffnet wurde (vgl. Klammernahtinstrumente).

Die Federkraft kann hierbei durch eine Blattfeder, eine Schenkelfeder oder eine Druckfeder aufgebracht werden. Bei einer Ausführung mit einer Blattfeder ist es insbesondere denkbar, die Elektrode mit Hilfe dieser Blattfeder zu kontaktieren.

Weiterhin wird vorgeschlagen, den Abklappmechanismus nicht durch das Herausziehen des Schafts zu realisieren, sondern durch das Vorschieben der Klinge auszulösen. Neben der nachfolgend dargestellten Lösung mit einer Doppelbohrung sind auch andere Ausführungen, z.B. mit einem Langloch, einer konisch zulaufenden Bohrung oder einer (anderen) Schnappverbindung denkbar. Die bereits vorgestellten Lösungen sind zudem sowohl für Klammernahtinstrumente als auch andere Instrumente zur Herstellung einer Anastomose denkbar und sind insbesondere in Bezug auf TFT anwendbar.

Eine bi-polare Auslegung ist genauso wie eine monopolare Auslegung, also einem Nutzen ohne Gegenelektrode zum zellenverbindenden Einwirken auf das Hohlorgan, möglich. Ein zirkuläres Versiegelungsinstrument mit besonders gutem Wirkungsgrad ist die Folge. Dabei kann die Elektrodenplatte selber als Amboss ausgebildet sein, der auch zur Umformung der Klammern herangezogen werden könnte. Bemerkenswert ist, dass bei der diesseitigen Ambosskonstruktion das Abwinkeln durch ein Herausziehen des Kopfstückschaftes ermöglicht ist. In Kombination mit der Doppelbohrung im verjüngten Bereich stellt das Konzept eine besonders effiziente Lösung für das besagte Problem bzgl. eines abklappbaren Ambosses bzw. einer abklappbaren Elektrodenplatte dar. Im Rahmen der vorliegenden Erfindung wird bei Nutzung des Anastomoseinstruments ausschließlich im Rahmen einer Hochfrequenzversiegelung das Gewebe nicht perforiert, sondern flächig und dicht versiegelt. Die Gefahr einer Leckage ist damit geringer, bzw. nahezu auszuschließen.

Der Abklappvorgang lässt sich ferner durch ein einfaches Einklicken des Kopfstückschafts, also das Erreichen einer Schnappverbindung wieder rückgängig machen. Der Querstift rastet dann in seiner ersten, proximalen Raststellung wieder ein. Dabei wird der besagte Formschluss zwischen dem Kopfstück und dem Kopfstückschaft wieder hergestellt. Dies kann von Vorteil sein, wenn die Elektrodenplatte zum leichteren Einführen abgewinkelt sein soll und erst dann in ihre Grundstellung, also die Bearbeitungsstellung, gebracht werden soll. Ein Beispiel hierfür sind minimalinvasive Eingriffe, etwa solche, bei denen die Elektrodenplatte transoral eingeführt wird. An dieser Stelle sei darauf hingewiesen, dass ein solcher Formschluss in einfacher Weise dadurch herstellbar ist, indem in der Elektrodenplatte/Amboss auf dessen Schaft-zugewandter Seite ein Axialloch oder eine Axialhülse ausgeformt ist, worin der Kopfstückschaft eingesteckt werden kann.

Der Abklappvorgang kann nicht nur durch das Herausziehen des Körperschafts ausgelöst werden, sondern alternativ oder zusätzlich (auch) durch das Hervorschieben einer Klinge, insbesondere einer kreisquerschnittaufweisenden Klinge bewirkt werden. Eine beim Abklappvorgang auftretende schälende Wirkung kann von Vorteil sein, falls es zu Gewebeanhaftungen zwischen den Elektroden kommt.

Die nachfolgend dargestellten Lösungen sind zudem insbesondere bei Klammernahtinstrumenten oder andere Instrumenten zur Herstellung einer Anastomose denkbar.

Unterschiedliche Ausführungsformen eines erfindungsgemäßen Anastomoseinstruments sind in den Zeichnungen dargestellt und werden nachstehend näher erläutert. Es zeigen:
Fig. 1 eine erste Ausführungsform eines Ausschnitts einer Amboss/Ambossbaugruppe eines Anastomoseinstruments in einem partiellen Längsschnitt in einer Bearbeitungsposition, unter Verwendung einer Litze zur Stromzuführung an eine Elektrode,
Fig. 2 einen Querschnitt durch den Kopfstückschaft der Ambossbaugruppe des Anastomoseinstruments aus Fig. 1 entlang der Linie I,
Fig. 3 der in Fig. 1 dargestellte Ausschnitt der Ambossbaugruppe des Anastomoseinstruments bei Vorhandensein des Kopfstücks in einer Entnahmeposition / Entnahmestellung (Litze und Blattfeder hier nicht dargestellt),
Fig. 4 eine zur Fig. 1 vergleichbare Darstellungsart einer zweiten Ausführungsform der Ambossbaugruppe eines Anastomoseinstruments, bei der eine Blattfeder zum direkten Kontaktieren der Elektrode eingesetzt ist und auf eine Litze verzichtet wird,
Fig. 5 eine dritte Ausführungsform der Ambossbaugruppe eines Anastomoseinstruments, das wie die in den Fig. 1 und 4 dargestellten Varianten in einer Bearbeitungsstellung wiedergegeben ist, wobei zwei Blattfedern eingesetzt werden und auf eine Litze verzichtet wird, und.
Fig. 6 eine vierte Ausführungsform der Ambossbaugruppe eines Anastomoseinstruments in einer Bearbeitungsstellung, wobei statt einer oder mehrerer Blattfedern eine Druckfeder eingesetzt wird, die in axialer Richtung wirkt, wobei zusätzlich eine Litze zum Kontaktieren der Elektrode vorgesehen ist.

Die Figuren sind lediglich schematischer Natur und dienen nur dem Verständnis der Erfindung. Die gleichen Elemente sind mit denselben Bezugszeichen versehen.

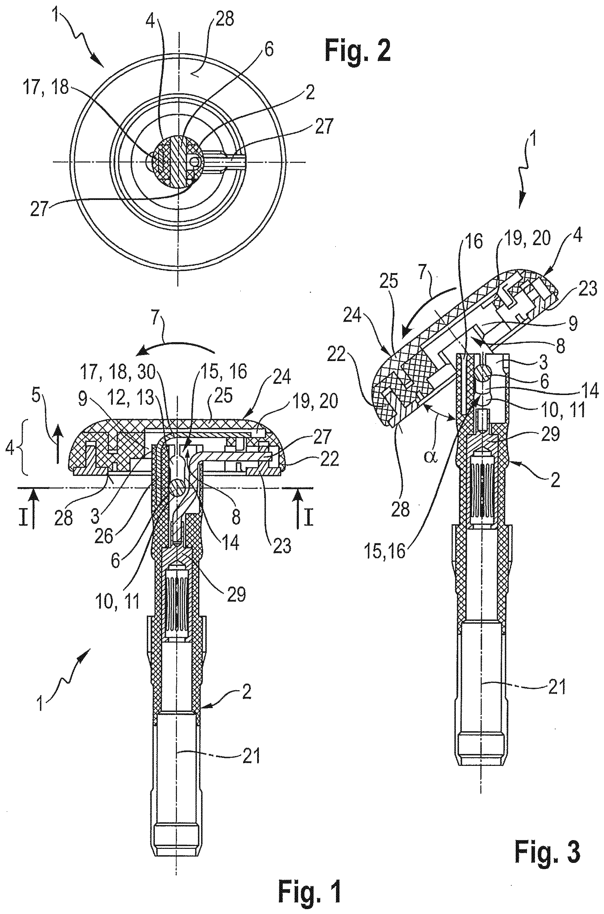

In Fig. 1 ist eine erste Ausführungsform einer erfindungsgemäßen Ambossbaugruppe 1 eines Anastomoseinstruments im Bereich von dessen distalem Endabschnitt dargestellt. Das Anastomoseinstrument hat demzufolge ein in einem nicht weiter dargestellten Instrumentenschaft an dessen distalem Ende axial verschieblich einsetzbares/eingesetztes Kopfstück 4. Insbesondere ist in dem Instrumentenschaft (an dessen distalem Ende), der an seinem gegenüberliegenden proximalen Ende auch ein Griffteil aufweisen kann, um einzelne Funktionseinheiten des Anastomoseinstruments zu betätigen, das Instrumentenkopfstück 4 axial verschieblich über einen eigenen Kopfstückschaft 2 eingesetzt. Der Kopfstückschaft 2 bildet zusammen mit dem Kopfstück 4 eine Art Pilz bestehend aus dem Schirm (Kopfstück 4) und dem Stil (Kopfstückschaft 2), die miteinander in Eingriff sind. Im Konkreten steckt ein distaler Endabschnitt 3 des Kopfstückschafts 2 in einer in Fig. 1 dargestellten Bearbeitungsposition in dem platten- oder ambossartigen Kopfstück 4 an dessen Unterseite. Das Kopfstück 4 ist dabei axial längs verschieblich (sowie umklappbar) am Kopfstückschaft 2 gelagert, wie dies nachfolgend im Einzelnen noch beschrieben wird. Das Kopfstück 4 kann in Gänze auch als Amboss bezeichnet werden. Das Kopfstück 4 ist entlang des Pfeiles 5 axial verschiebbar im Kopfstückschaft 3 über eine nachfolgend beschriebene Mechanik gehalten.

Das Kopfstück 4 weist an seiner der distalen Stirnseite des Kopfstückschafts 2 zugewandten (flachen) Unterseite eine axial vorragende Halterung/Schwenklagerung auf (in den Figuren nicht näher dargestellt), in der ein Quer - Schwenkbolzen oder Querstift 6 aufgenommen ist. Der Querstift 6 ordnet sich somit im Wesentlichen parallel zur Unterseite des Kopfstücks 4 sowie im Abstand hierzu an.

An der Unterseite des Kopfstücks 4 sowie im Höhenabstand zum Querstift 6 (d.h. unmittelbar oberhalb des Querstifts 6') ist eine Art Aufnahme 8 für den distalen Endabschnitt 3 des Kopfstückschafts 4 ausgebildet, in die der Endabschnitt 3 axial verschieblich aber unverschwenkbar eingeführt/eingesteckt werden kann.

Ist / wird demnach das Kopfstück 4 in Richtung des Pfeiles 5 distal vom Kopfstückschaft 2 wegbewegt, sodass dessen distaler Endabschnitt aus der Aufnahme gleitet, so kann es bei entsprechend ausreichendem Abstand um den Querstift 6 in Richtung des Pfeiles 7 verschwenken. Das Kopfstück 4 gelangt dann in die in Fig. 3 dargestellte Entnahmeposition.

In der Bearbeitungsposition herrscht zwischen der radialen Außenseite des distalen Endabschnitts 3 und einer Innenseite der Aufnahme oder Ausnehmung 8 im Bereich einer an der Aufnahme 8 ausgebildeten Schulter 9 ein Formschluss, der das Kopfstück 4 vor einer Schwenkbewegung in Richtung des Pfeiles 7 bewahrt bzw. es daran hindert. Das Kopfstück 4 kann in der Ausnehmung 8 eine (Axial-)Verzahnung aufweisen, wie ein Keilprofil. Das distale Ende 3 des Kopfstückschafts 2, also das dem Kopfstück 4 zugewandte Ende, kann eine dazu passende Außenverzahnung aufweisen, etwa nach Art einer Keilwelle. Evolventenartige Flankengeometrien sind ebenfalls möglich.

Dabei befindet sich der Querstift 6 in einer Kopfstückschaft - seitigen (Quer-) Öffnung 10, die als eine erste Quer - Durchgangsbohrung 11 an distalen Endabschnitt 3 ausgestaltet sein kann. Diese erste Querbohrung 11 ist von der distalen Stirnseite des Kopfstückschafts 3 weiter entfernt, als eine weitere (Quer-) Öffnung 12, die als eine zweite Quer - Durchgangsbohrung 13 ausgebildet ist. Die erste Querbohrung 11 und die zweite Querbohrung 13 erstrecken sich parallel zueinander sowie orthogonal zur Längsachse 21 des Kopfstückschafts 2 komplett durch den Kopfstückschaft 2 hindurch.

Die beiden Öffnungen 10 und 12 sind über einen Verjüngungsbereich / Dehnungsbereich 14 voneinander beabstandet. Diese beiden Öffnungen 10 und 12, also die erste und zweite Querbohrung 11 und 13 sowie der Verjüngungsbereich / Bereich erhöhten Widerstands / Dehnungsbereich 14 sind Teil einer inneren (Kulissen-) Führung 15, die vorliegend als gerader Längsschlitz 16 am distalen Endabschnitt 3 des Kopfstückschafts 2 ausgebildet ist. Der Längsschlitz 16 erstreckt sich bis zur distalen Stirnseite, teilt also den Kopfstückschaft 2 dort regelrecht in zwei federelastisch voneinander entfernbare Teilhohlzylinder (Schaft - Längsgabeln), wobei die erste Querbohrung 11 den proximalen Abschluss des Längsschlitzes 16 bildet und die zweite Querbohrung 12 axial vor der distalen Stirnseite des Kopfstückschafts 2 und damit vor dem distalen Ende des Längsschlitzes 16 platziert ist. Der Längsschlitz 16 kann gerade oder krümmt ausgeformt sein oder gekrümmte Abschnitte aufweisen. Er kann auch einer Schraubenform nachempfunden sein. Der Längsschlitz muss sich nicht zwangsläufig bis zum distalen Ende des Kopfstückschafts erstrecken.

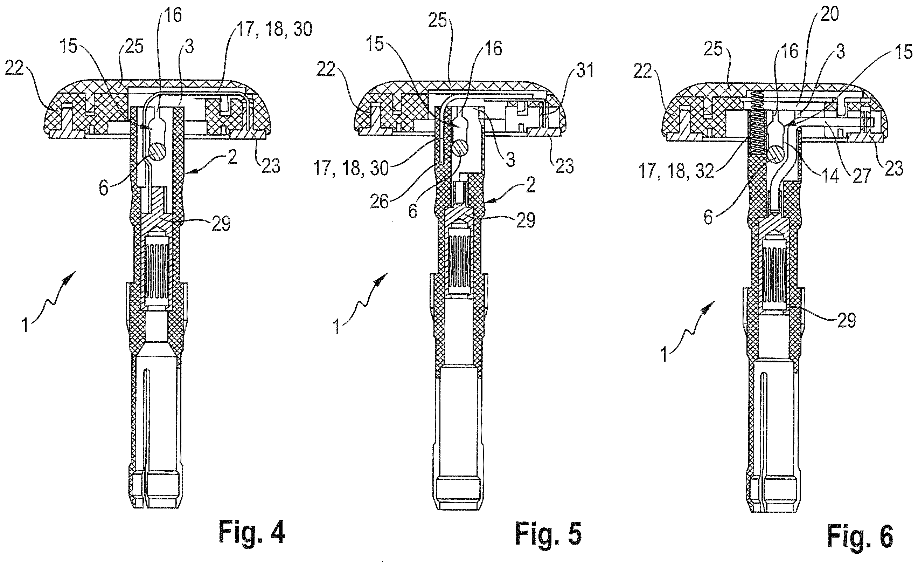

Ein Federelement 17 ist nach Art einer mechanischen Feder 18 ausgebildet, insbesondere hier als (im entspannten Zustand gerade) Blattfeder, z.B. als Blattfeder 30 (siehe Fign. 1, 4 und 5) oder 31 (siehe Fig. 5).

Die in Fig. 1 dargestellte Blattfeder 30 steckt unverschieblich in einer in der Wandung des Kopfstückschafts 2 ausgebildeten Axialnut und erstreckt sich in eine Ausnehmung oder Nut 19, die nach Art eines Hohlraumes 20 ausgebildet ist, in das Kopfstück 4 hinein. Wie es in der Fig. 1 dargestellt ist (also in der Bearbeitungsposition), ist die Ausnehmung 19 bzw. der Hohlraum 20 dabei in dieser Lage des Kopfstücks 4 quer zu einer Längsachse 21 des Kopfstückschafts 2 ausgerichtet, insbesondere rechtwinklig dazu ausgerichtet. Die mechanische Feder 30 ist dabei vorgespannt (elastisch gebogen).

Das Kopfstück 4 weist an seiner Unterseite gemäß vorstehender Definition in der dargestellten Ausführungsform eine Elektrodenplatte 22 auf, in der eine oder mehrere Elektroden 23 (vorzugsweise unverschieblich) gehalten sind. Auf der distalen Seite 24 des Kopfstücks 4 ist eine pilzförmige Abdeckkappe 25 angeordnet, welche die Elektrodenplatte oberseitig abdeckt.

Der Hohlraum 20 befindet / bildet sich zwischen der Elektrodenplatte 22 und der Abdeckkappe 25, wobei sich die darin befindliche Feder 30 in der Bearbeitungsposition um 90° elastisch verbiegt. Ein proximales Ende 26 der Feder 30 ist dezentral, also quer beabstandet zur Längsachse 21 im Kopfstückschaft 2 form- und/oder kraftschlüssig in die Axialnut eingesetzt. D.h. die eine Schwenkkraft ausübende Feder 30 ist dezentral zum Schwenkstift 6 angeordnet. Im Hohlraum 20 ist die Feder 18 daher nicht (positionsfest) fixiert, sondern verschiebbar gehalten, um im Fall einer Verschwenkung des Kopfstücks 4 um den Schwenkstift 6 eine axiale Kompensations-Relativverschiebung zwischen Kopfstück 4 / Hohlraum 20 und Feder 30 zu ermöglichen.

Während die Elektrode 23 mehrere einzelne Segmente umfassen kann, die ringartig ausgebildet sind oder die Elektrode 23 als durchgehender Ring ausgebildet sein kann, ist es möglich, dass zusätzlich oder anstelle der Elektrode 23 nicht dargestellte Taschen aufweisende Einsätze vorhanden sind, mit Hilfe derer Klammern umbiegbar sind. Auch kann die Elektrode 23 entsprechend ausgeformt sein. Diese Elemente können zusätzlich unter Strom gesetzt werden. Im vorliegenden Ausführungsbeispiel wird die Stromzufuhr zu der Elektrode 23 über eine Litze 27 bewirkt.

Wird das Kopfstück 4 durch bewusstes Verschieben des Querstifts 6 in Richtung des Pfeiles 5 vom Kopfstückschaft 2 wegbewegt, etwa ausschließlich oder zusätzlich unter Nutzung einer nicht dargestellten Klinge, wie einer Kreisklinge, welche im distalen Endabschnitt des nicht weiter gezeigten Instrumentenschafts axialverschiebbar gelagert ist, so bewegt sich der Querstift 6, der vorzugsweise axial unverschieblich im Kopfstück 4 in der Halterung gelagert ist, aus der ersten Querbohrung 11 heraus, und überwindet dabei den Dehnungsbereich 14 bzw. den durch diesen erzeugten Federwiderstand, vorausgesetzt, dass eine ausreichend große Schiebekraft auf das Kopfstück 4 aufgebracht wird - und gelangt erst dann (erneut verrastend) in die zweite Querbohrung 13. Der Querstift 6 ist demzufolge je nach eingenommener Position in einer der beiden Querbohrungen 11 und 13 verrastet.

Ist der Querstift 6 in der ersten Querbohrung 11 aufgenommen, so befindet sich das Kopfstück 4 in der ersten Raststellung, in welcher der distale Endabschnitt 3 des Kopfstückschaft 2 in der unterseitigen Ausnehmung des Kopfstücks 3 formschlüssig eingesteckt ist. Ist der Querstift 6 in der zweiten Querbohrung 13 vorhanden, befindet sich das Kopfstück 4 in der zweiten Raststellung. Ist diese zweite Raststellung eingenommen, so ist der Formschluss insbesondere zwischen der Schulter 9 der kopfstückseitigen Ausnehmung und dem distalen Endabschnitt 3 aufgehoben und die bis zu diesem Zeitpunkt vorgespannte mechanische Feder 30 kann dann selbsttätig, d.h. automatisch, das Kopfstück 4 in die in Fig. 4 dargestellte Entnahmeposition um den Querstift 6 verschwenken.

Wie in Fig. 3 zu erkennen ist, stellt sich dabei zwischen der Unterseite 28 des Kopfstücks 4 und der Längsachse 21 ein Winkel α ein, der vorzugsweise im Bereich von 2,5° - 45° liegen kann. Eine Kontaktbuchse 29, die mit der Litze 27 verbunden ist, ist in das Innere des Kopfstückschafts 2, proximal vom distalen Endabschnitt 3 beabstandet, eingesetzt. Während in Fig. 3 der Zustand der Ambossgruppe 1 des Anastomoseinstruments in der Entnahmeposition dargestellt ist, sei darauf hingewiesen, dass dort das komplette Federelement 17, also die mechanische Feder 30, die als Blattfeder ausgebildet ist, nicht dargestellt ist. Sie ist aber natürlich auch in der Entnahmeposition vorhanden.

In den Fig. 4 bis 6 sind die Bearbeitungspositionen dreier weiterer, alternativer, aber erfindungsgemäßer Ambossbaugruppen 1 eines Anastomoseinstruments dargestellt. Der Aufbau entspricht in großen Zügen dem Aufbau des in den Fig. 1 bis 3 dargestellten ersten Ausführungsbeispiels des Anastomoseinstruments. Die diesbezüglichen Details werden hier nicht wiederholt, es sei aber auf sie verwiesen.

Einige Unterschiede zum ersten Ausführungsbeispiel sollen jedoch herausgestellt werden: So wird in den Ausführungsbeispielen der Fig. 4 und 5 wieder auf die Verwendung eines mechanischen Federelementes 17, also einer mechanischen Feder 18 abgestellt. In den beiden Fällen der Ausführungsbeispiele der Fig. 4 und 5 sind Blattfedern eingesetzt. Es sei jedoch darauf hingewiesen, dass in dem Ausführungsbeispiel der Fig. 5 zwei Blattfedern eingesetzt werden und auf eine Litze gemäß dem ersten Ausführungsbeispiel, welche die Energieversorgung der Elektrode(n) bereitstellt, verzichtet ist. Auf diese Litze ist auch in dem in Fig. 4 dargestellten Ausführungsbeispiel verzichtet.

Die blattfederartige mechanische Feder 18 kann in diesem Fall an einem Einsatz 29, der im Kopfstückschaft befindlich ist, angebracht sein und mit der Elektrode 23 in (elektrischem) Kontakt stehen, wie dies insbesondere in Fig. 4 dargestellt ist.

Eine erste Blattfeder 30, wie sie auch im Ausführungsbeispiel gemäß Fig. 1 dargestellt wird, ist mit einem proximalen Ende 26 in dem Kopfstückschaft 2 fest eingesetzt. Sie ist jedoch auch in Anlage mit einer zweiten Blattfeder oder einem anderen Leiterbauteil 31, das dann, wie im Ausführungsbeispiel gemäß Fig. 5 gezeigt ist, mit der Elektrode 23 in elektrischem Kontakt steht.

Da die beiden Ausführungsbeispiele der Fig. 4 und 5 auf Litzen parallel zur Feder verzichten, erfolgt die elektrische Kontaktierung der Elektrode(n) 23 ausschließlich über die (Blatt-) Feder(n), also im Falle des Ausführungsbeispiels gemäß Fig. 4 über eine einzige Blattfeder 30 und im Ausführungsbeispiel gemäß Fig. 5 beispielsweise über die zwei Blattfedern 30 und 31 (oder über die Feder 30 und einem daran anliegenden Leiterbauteil 31), welche miteinander in verschiebbarem Kontakt stehen.

Grundsätzlich ist auch hier wiederum ein kopfstückseitiger Querstift 6 in einer kopfstückschaftseitigen, durch einen Längsschlitz 16 gebildeten (Kulissen-) Führung 15 befindlich, es kann jedoch auch auf konische Bohrungen zurückgegriffen werden oder (alternative) Schnappverbindungen vorgesehen werden.

In den vorstehen genannten Fällen erfolgt der Einsatz der erfindungsgemäßen Kopfstück - Kopfstückschaft - Konstruktion (Ambossbaugruppe 1) in einem zirkulären Versiegelungselement als das Anastomoseinstrument, unter Vermeidung von Klammern.

Das gilt auch für das in der Fig. 6 dargestellte Ausführungsbeispiel, in welchem jedoch anderes als in den vorher diskutierten Ausführungsbeispielen eine Druckfeder, nämlich eine Schraubenfeder 32 in einem dezentral, d.h. exzentrischen (Axial-) Loch 33 angeordnet ist und über die distale Stirnseite des Kopfstückschafts 2 hinaus in den Hohlraum 20 des Kopfstücks 2 ragt sowie vorzugsweise in axialer Anlage mit einem distalen Abschnitt des Kopfstücks 4, vorzugsweise der Abdeckkappe 25 befindlich ist. Die Kontaktierung der Elektrode 23 findet wiederum über eine Litze 27 statt, wie schon bzgl. des Ausführungsbeispiels der Fig. 1 bis 3 diskutiert wurde.

Auch hier, wie in allen vorhergehenden Ausführungsbeispielen, wird ein kopfstückseitiger Querstift 6 in einer kopfstückschaftseitigen, einen Längsschlitz 16 bildenden Führung 15, zwischen zwei axial beabstandeten Raststellungen unter Überwindung eines durch einen Verjüngungsbereich 14 im Kopfstückschaft erzeugten Federwiderstands hin und her bewegbar, eingesetzt. Alternativ kann jedoch auch eine Kugel-Feder-Kombination genutzt werden, wobei der Verjüngungsbereich durch eine senkrecht zur Schaftachse verschiebbare sowie federvorgespannte Kugel ausgebildet wird. Weiter alternativ kann eine einen Druckpunkt überwindende Ausgestaltung eines Rastmechanismus umgesetzt werden.

### Bezugszeichenliste

- 1: Amboss / Ambossbaugruppe
- 2: Kopfstückschaft
- 3: distaler Endabschnitt
- 4: Kopfstück/Amboss
- 5: Pfeil
- 6: Querstift
- 7: Pfeil
- 8: Ausnehmung
- 9: Schulter
- 10: Öffnung
- 11: erste Querbohrung
- 12: Öffnung
- 13: zweite Querbohrung
- 14: Verjüngungsbereich
- 15: Führung
- 16: Längsschlitz
- 17: Federelement
- 18: mechanische Feder
- 19: Ausnehmung
- 20: Hohlraum
- 21: Längsachse
- 22: Elektrodenplatte
- 23: Elektrode
- 24: distales Ende des Kopfstücks
- 25: Abdeckkappe
- 26: proximales Ende der Feder
- 27: Litze
- 28: Unterseite
- 29: Kontaktbuchse
- 30: erste Blattfeder
- 31: zweite Blattfeder
- 32: Schraubenfeder

## Patentansprüche

1. Anastomoseinstrument mit einem in einem Instrumentenschaft axial verschiebbar einführbaren Kopfstückschaft (2), an dessen distalen Endabschnitt (3) ein platten- und/oder ambossartiges Kopfstück (4) axial verschiebbar und über einen Querstift (6) zwischen einer Bearbeitungsposition und einer Entnahmeposition schwenkbar gelagert ist, wobei das Kopfstück (4) am Kopfstückschaft (2) aus einer ersten, proximal zurückgeschobenen Raststellung, in welcher der Kopfstückschaft (2) formschlüssig mit dem Kopfstück (4) gekoppelt ist, um das Kopfstück (4) in der Bearbeitungsposition unverschwenkbar zu fixieren, in eine zweite, distal vorgeschobene Raststellung verlagert oder verschoben ist, in welcher der Formschluss freigegeben ist, um ein Verschwenken des Kopfstücks (4) in die Entnahmeposition zu ermöglichen, **dadurch gekennzeichnet, dass** der kopfstückseitige Querstift (6) in einem axial sich erstreckenden Längsschlitz (16) zwischen den beiden Raststellungen verschieblich, hin und her bewegbar geführt ist, wobei der Längsschlitz (16) an einem distalen Endabschnitt des Kopfstückschafts (2) ausgeformt ist.

2. Anastomoseinstrument nach Anspruch 1, **dadurch gekennzeichnet, dass** das Kopfstück (4) auf seiner proximalen, dem Kopfstückschaft (2) zugewandten Seite eine Elektrode (23) aufweist.

3. Anastomoseinstrument nach Anspruch 1 oder 2, **dadurch gekennzeichnet, dass** der Kopfstückschaft (2) im Bereich des Längsschlitzes (16) in dessen Querrichtung federelastisch ausgebildet ist, um einen Federwiderstand auf den Querstift (6) auszuüben, wenn dieser aus seiner Bearbeitungsposition in die Entnahmeposition entlang des Längsschlitzes (16) verschoben wird.

4. Anastomoseinstrument nach einem der Ansprüche 1 bis 3, **dadurch gekennzeichnet, dass** das Kopfstück (4) in der zweiten, distal vorgeschobenen Raststellung um den Querstift (6) in die Entnahmeposition verschwenkbar ist.

5. Anastomoseinstrumentnach einem der Ansprüche 1 bis 4, **dadurch gekennzeichnet, dass** ein Federelement (17), wie eine mechanische Feder (18), so am Kopfstück (4) und am Kopfstückschaft (2) angebracht ist, dass es ein Verschwenken des Kopfstücks (4) bei Verlagerung des Kopfstücks (4) von der ersten Raststellung in die zweite Raststellung erzwingt.

6. Anastomoseinstrument nach Anspruch 5, **dadurch gekennzeichnet, dass** die Feder (18) dezentral, von der Längsachse (21) des Kopfstückschafts (2) quer beabstandet im Kopfstückschaft (2) gelagert ist.

7. Anastomoseinstrument nach Anspruch 5, **dadurch gekennzeichnet, dass** die Feder (18) zentral und der Längsschlitz (16) dezentral von der Längsachse (21) des Kopfstückschafts (2) platziert sind.

8. Anastomoseinstrument nach Anspruch 5, 6 oder 7, **dadurch gekennzeichnet, dass** die Feder (18) im Kopfstück (4) oder im Kopfstückschaft (2) verschieblich gelagert ist, vorzugsweise in einer nutenartigen Ausnehmung (19).

9. Anastomoseinstrument nach einem der Ansprüche 5 bis 8, **dadurch gekennzeichnet, dass** die Feder (18) als Blattfeder, Schenkelfeder, Zug- oder Druckfeder, ausgebildet ist.

10. Anastomoseinstrument nach einem der Ansprüche 1 bis 9, **dadurch gekennzeichnet, dass** beim Verbringen des Querstifts (6) von der ersten Raststellung in die zweite Raststellung im Längsschlitz (16) des Kopfstücks (4) eine vorbestimmte Widerstandskraft zu überwinden ist, die größer ist als die zu erwartenden maximalen Arbeitskräfte, die in axialer Richtung auf das Kopfstück (4) während einer chirurgischen Behandlung einwirken.

11. Anastomoseinstrument nach einem der Ansprüche 1 bis 9, **dadurch gekennzeichnet, dass** die Aktivierungskraft zum Auslösen der ersten Raststellung kleiner ist als die bei der Gewebebehandlung zu erwartenden maximalen Arbeitskräfte.

12. Anastomoseinstrument nach einem der Ansprüche 1 bis 11, **dadurch gekennzeichnet, dass** der Formschluss durch eine axiale Ausnehmung oder Vorsprung am Kopfstück (4) gebildet ist, welche an dessen dem Kopfstückschaft (2) zugewandten Unterseite ausgeformt ist und in welche der distale Endabschnitt (3) des Kopfstückschafts (2) in der Bearbeitungsposition schwenkfest eingreift.

13. Anastomoseinstrument nach einem der vorstehenden Ansprüche, **dadurch gekennzeichnet, dass** es ein TFT- oder ein Klammerinstrument ist.

## Claims

1. Anastomosis instrument with a headpiece shaft (2) which can be inserted in an instrument shaft so as to be axially shiftable therein, a plate-like and/or anvil-like headpiece (4) being mounted on a distal end portion (3) of said headpiece shaft so as to be axially shiftable and supported, via a cross pin (6), so as to pivot between a processing position and a withdrawal position, wherein the headpiece (4) is displaceable or shiftable on the headpiece shaft (2) from a first, proximally retracted latching position in which the headpiece shaft (2) is coupled to the headpiece (4) in a form-fitting manner to fix the headpiece (4) in the processing position so that it is unable to pivot, to a second, distally advanced latching position in which the form fit is released in order to allow the headpiece (4) to pivot to the withdrawal position, **characterised in that** the cross pin (6) at the headpiece is movably guided in an axially extending longitudinal slit (16) so as to be able to be moved back and forth between the two latching positions, wherein the longitudinal slit (16) is formed on a distal end portion of the headpiece shaft (2).

2. Anastomosis instrument according to claim 1, **characterised in that** the headpiece (4) comprises an electrode (23) on its proximal side facing the headpiece shaft (2).

3. Anastomosis instrument according to claim 1 or 2, **characterised in that** the headpiece shaft (2) is formed to be resilient in the area of the longitudinal slit (16) in its transverse direction in order to exert a spring resistance on the cross pin (6) if the latter is shifted from its processing position to the withdrawal position along the longitudinal slit (16).

4. Anastomosis instrument according to any of claims 1 to 3, **characterised in that** in the second, distally advanced latching position the headpiece (4) can be pivoted around the cross pin (6) into the withdrawal position.

5. Anastomosis instrument according to any of claims 1 to 4, **characterised in that** a spring element (17), such as a mechanical spring (18), is attached to the headpiece (4) and the headpiece shaft (2) in such a manner that it enforces a pivoting of the headpiece (4) when the headpiece (4) is displaced from the first latching position to the second latching position.

6. Anastomosis instrument according to claim 5, **characterised in that** the spring (18) is eccentrically supported in the headpiece shaft (2) so as to be transversely spaced from the longitudinal axis (21) of the headpiece shaft (2).

7. Anastomosis instrument according to claim 5, **characterised in that** the spring (18) is placed centrically and the longitudinal slit (16) is placed off-center relative to the longitudinal axis (21) of the headpiece shaft (2).

8. Anastomosis instrument according to claim 5, 6 or 7, **characterised in that** the spring (18) is shiftably supported in the headpiece (4) or in the headpiece shaft (2), preferably in a groove-like recess (19).

9. Anastomosis instrument according to any of claims 5 to 8, **characterised in that** the spring (18) is formed as a leaf spring, leg spring, tension spring or compression spring.

10. Anastomosis instrument according to any of claims 1 to 9, **characterised in that** a predetermined resisting force has to be overcome during a transfer of the cross pin (6) from the first latching position to the second latching position in the longitudinal slit (16) of the headpiece (4), which is larger than the expectable maximum working forces acting onto the headpiece (4) in the axial direction during a surgical treatment.

11. Anastomosis instrument according to any of claims 1 to 9, **characterised in that** the activation force for triggering the first latching position is smaller than the maximum working forces which are to be expected during the tissue treatment.

12. Anastomosis instrument according to any of claims 1 to 11, **characterised in that** the form fit is formed by an axial recess or protrusion on the headpiece (4), the recess or protrusion being shaped on its underside facing the headpiece shaft (2) and in which the distal end portion (3) of the headpiece shaft (2) engages in a pivot-proof manner in the processing position.

13. Anastomosis instrument according to any of the preceding claims, **characterised in that** it is a TFT instrument or a clip setting instrument.

## Revendications

1. Instrument d'anastomose comprenant une tige de pièce de tête (2) insérable par coulissement axial dans une queue d'instrument, à la section d'extrémité distale (3) de laquelle tige une pièce de tête (4) de type plaque et/ou enclume est montée à coulissement axial et peut pivoter par le biais d'une tige transversale (6) entre une position de traitement et une position de retrait, dans lequel la pièce de tête (4) est décalée ou déplacée sur la tige de pièce de tête (2) d'une première position d'encliquetage reculée en direction proximale, dans laquelle la tige de pièce de tête (2) est couplée par complémentarité de formes à la pièce de tête (4) pour fixer la pièce de tête (4) en position de traitement sans pouvoir pivoter, à une seconde position d'encliquetage avancée en direction distale, dans laquelle la liaison à complémentarité de formes est libérée pour permettre un pivotement de la pièce de tête (4) en position de retrait, **caractérisé en ce que** la tige transversale côté pièce de tête (6) est guidée de façon mobile en va-et-vient dans une fente longitudinale (16) s'étendant axialement de manière à pouvoir se déplacer entre les deux positions d'encliquetage, dans lequel la fente longitudinale (16) est ménagée sur une section d'extrémité distale de la tige de pièce de tête (2).

2. Instrument d'anastomose selon la revendication 1, **caractérisé en ce que** la pièce de tête (4) présente une électrode (23) sur son côté proximal tourné vers la tige de pièce de tête (2).

3. Instrument d'anastomose selon la revendication 1 ou 2, **caractérisé en ce que** la tige de pièce de tête (2) est formée de manière élastique dans la zone de la fente longitudinale (16) dans son sens transversal pour exercer une résistance élastique sur la tige transversale (6) lorsque celle-ci est déplacée de sa position de traitement à la position de retrait le long de la fente longitudinale (16).

4. Instrument d'anastomose selon l'une quelconque des revendications 1 à 3, **caractérisé en ce que** la pièce de tête (4) peut pivoter en position de retrait autour de la tige transversale (6) dans la seconde position d'encliquetage avancée en direction distale.

5. Instrument d'anastomose selon l'une quelconque des revendications 1 à 4, **caractérisé en ce qu'**un élément de ressort (17), comme un ressort mécanique (18), est monté sur la pièce de tête (4) et sur la tige de pièce de tête (2) de sorte qu'il provoque un pivotement de la pièce de tête (4) lors du déplacement de la pièce de tête (4) de la première position d'encliquetage à la seconde position d'encliquetage.

6. Instrument d'anastomose selon la revendication 5, **caractérisé en ce que** le ressort (18) est logé dans la tige de pièce de tête (2) de manière décentralisée à distance transversale de l'axe longitudinal (21) de la tige de pièce de tête (2).

7. Instrument d'anastomose selon la revendication 5, **caractérisé en ce que** le ressort (18) est placé de manière centralisée et la fente longitudinale (16) de manière décentralisée par rapport à l'axe longitudinal (21) de la tige de pièce de tête (2).

8. Instrument d'anastomose selon la revendication 5, 6 ou 7, **caractérisé en ce que** le ressort (18) est logé à coulissement dans la pièce de tête (4) ou dans la tige de pièce de tête (2), de préférence dans une cavité en forme de rainure (19).

9. Instrument d'anastomose selon l'une quelconque des revendications 5 à 8, **caractérisé en ce que** le ressort (18) se présente sous la forme d'un ressort à lames, d'un ressort spiralé, d'un ressort de traction ou de compression.

10. Instrument d'anastomose selon l'une quelconque des revendications 1 à 9, **caractérisé en ce que,** lors du transfert de la tige transversale (6) de la première position d'encliquetage à la seconde position d'encliquetage dans la fente longitudinale (16) de la pièce de tête (4), il faut surmonter une force de résistance prédéterminée qui est supérieure aux forces de travail maximales à escompter, qui agissent dans la direction axiale sur la pièce de tête (4) au cours d'un traitement chirurgical.

11. Instrument d'anastomose selon l'une quelconque des revendications 1 à 9, **caractérisé en ce que** la force d'activation pour déclencher la première position d'encliquetage est inférieure aux forces de travail maximales à escompter lors du traitement des tissus.

12. Instrument d'anastomose selon l'une quelconque des revendications 1 à 11, **caractérisé en ce que** la liaison à complémentarité de formes est formée par une cavité ou une saillie axiale sur la pièce de tête (4), qui est formée sur la face inférieure tournée vers la tige de pièce de tête (2) et dans laquelle la section d'extrémité distale (3) de la tige de pièce de tête (2) vient en prise dans la position de traitement en résistant au pivotement.

13. Instrument d'anastomose selon l'une quelconque des revendications précédentes, **caractérisé en ce qu'**il s'agit d'un instrument TFT ou d'un instrument à griffes.
